# EUROPÄISCHE PATENTSCHRIFT

(11) **EP 1 079 897 B1**
(45) Veröffentlichungstag und Bekanntmachung des Hinweises auf die Patenterteilung: **17.11.2004**
(21) Anmeldenummer: 99953301.1
(22) Anmeldetag: 28.05.1999
(51) Int. Cl.: A61N 5/06

(54) **VORRICHTUNG ZUM OBERFLÄCHLICHEN ERWÄRMEN VON GEWEBE**
DEVICE FOR THE SUPERFICIAL HEATING OF TISSUE
DISPOSITIF POUR CHAUFFER SUPERFICIELLEMENT UN TISSU

(30) Priorität: 28.05.1998 DE 19823947
(43) Veröffentlichungstag der Anmeldung: 07.03.2001
(73) Patentinhaber: Carl Baasel Lasertechnik GmbH, D-82319 Starnberg (DE)
(72) Erfinder: HIBST, Raimund, D-89155 Erbach (DE); FALKENSTEIN, Werner, D-82319 Starnberg (DE)
(74) Vertreter: Feldkamp, Rainer, Dipl.-Ing.
(86) Internationale Anmeldenummer: PCT/EP1999/003720
(87) Internationale Veröffentlichungsnummer: WO 1999/061105

(56) Entgegenhaltungen:
- EP-A- 0 763 371
- WO-A-97/37723
- WO-A-98/24514

## Beschreibung

Die Erfindung bezieht sich auf eine Vorrichtung zum oberflächlichen Erwärmen von Gewebe der im Oberbegriff des Patentanspruchs 1 genannten Art.

Zur Beseitigung von Falten und zur Glättung vorgealterter Haut (z.B. durch übermäßige Sonneneinstrahlung) werden in der Dermatologie alternativ zum chemischen Peeling seit einiger Zeit erfolgreich Lasersysteme wie der Er:Yag- und CO₂-Laser eingesetzt. Mit diesen Lasern wird die Haut oberflächlich abgetragen. Ähnlich wie beim Peeling steigen mit der Abtragtiefe - von ca. 60µm (superfizieller Abtrag) bis zu 0,8 mm (tiefes Peeling) - sowohl die glättende Wirkung als auch die Wahrscheinlichkeit unerwünschter Nebenwirkungen (Narben, Hyper- oder Hypopigmentierung) an. Durch den Abtrag kann die Hautoberfläche zum Teil planiert werden, außerdem wird eine Straffung beobachtet. Nach dem derzeitigen Stand der Literatur wird die hautstraffende Wirkung dadurch erklärt, daß geschädigte, elastotische Hautschichten entfernt und im Verlauf der anschließenden Wundheilung durch eine Reparaturschicht mit neuem Kollagen vom Typ I ersetzt werden. Dieser Effekt ist im Prinzip davon unabhängig, auf welche Weise die Haut abgetragen oder irreversibel verändert wurde, er erklärt, warum mechanische Dermabrasion, chemische Agenzien oder laserinduzierte Ablationen gleichermaßen wirksam sind.

Als weiterer, nur beim Laser vorhandener, Wirkungsmechanismus wird die wärmeinduzierte Schrumpfung von Kollagen diskutiert. Typ I Kollagen-Fibrillen schrumpfen bei Erwärmung auf einen Temperaturbereich zwischen 55°C bis 60°C und 70°C (Beginn der Koagulation) bis zu einem Drittel ihrer Länge, ohne biologisch inaktiv zu werden. Dies ist ein unmittelbar mit der Erwärmung einsetzender Prozeß. Fitzpatrick et al. vermuten diesen Effekt als Ursache einer beim "Resurfacing" unerwartet beobachteten sofortigen Straffung loser und gefalteter Haut. (R.E. Fitzpatrick et al.: Pulsed Carbon Dioxide Laser Resurfacing of Photoaged Facial Skin, Arch Dermatol 132, 395-402, 1996).

Das auf einem Abtrag der Haut basierende "Resurfacing" ist zwar in vielen Fällen wirkungsvoll, aber auch mit negativen Begleiterscheinungen verbunden. Die wichtigsten sind:
- postoperative Infektionsgefahr (zum Teil mehrtägige Abdeckung des ganzen Gesichtes mit Folie gefordert)
- mehrere Wochen lang anhaltendes Erythem
- eingeschränkte soziale Verkehrsfähigkeit für mindestens eine Woche
- mögliche Hyperpigmentierung, seltener Hypopigmentierung

Außerdem zeigt die neugebildete Haut in Bezug auf ihre Struktur Abweichungen zur übrigen Haut.

Bei der bisherigen Faltenglättung mit Hilfe von CO₂- und Er:YAG-Lasern spielt für das Resultat vermutlich der Abtrag und die Neubildung der Haut die Hauptrolle. Diese Hypothese wird durch die Tiefenausdehnung der behandelten Hautschichten plausibel: dem Mechanismus der Hautneubildung sind die Regionen der abgetragenen (etwa 100 µm) und der irreversibel geschädigten, koagulierten (Co₂Laser ca. 80 bis 100 µm, Er:YAG-Laser ca. 40 µm) Haut unterworfen. Für die Kollagenschrumpfung steht hingegen nur der an die Koagulationszone angrenzende Bereich zur Verfügung, der im Temperaturintervall zwischen der Koagulationstemperatur von etwa 70°C und der Untergrenze der Kollagenschrumpfungstemperatur von etwa 55°C bis 60°C liegt. Dieser Bereich ist beim Hautabtrag für beide Laser in seiner Tiefenausdehnung verhältnismäßig dünn, so daß man einen nennenswerten Beitrag zur Glättung nicht erwarten kann, wiewohl er bei CO₂-Lasern größer als bei z.B. Er:YAG-Lasern eingeschätzt wird.

Aus der WO-A-97 37723 ist eine Vorrichtung der eingangs genannten Art bekannt, bei dem bzw. bei der die Laserwellenlänge bezogen auf die Dicke des Zielgewebes und den spektralen Absorptionskoeffizienten dieser Wellenlänge so gewählt wird, daß die Laserstrahlung die Kollagenschicht in der Tiefe der Haut erreicht, ohne in den darüberliegenden Hautschichten eine wesentliche Absorption zu erfahren. Die Hautoberfläche kann hierbei vor der Aufbringung der Laserstrahlung gekühlt werden, um eine Beeinträchtigung der Hautoberfläche durch die mögliche geringe Absorption in der Oberhaut zu vermeiden. Hierbei ist die Auswahl des Lasers wegen der erforderlichen hohen Eindringtiefe in das Gewebe auf bestimmte Laserwellenlängen beschränkt.

Aus der EP-A2-0 763 371 ist ein Verfahren bzw. eine Vorrichtung zur Hautverjüngung und Faltenglättung bekannt, bei der eine Ablation durch einen Er:YAG-Laser erfolgt, wobei zusätzlich eine Blitzlampe Lichtimpulse im Bereich von 600-1000 nm zur Aufwärmung des Collagens verwendet wird. Der Er:YAG-Laser bewirkt lediglich eine Ablation der Oberfläche aufgrund der geringen Eindringtiefe der Lichtimpulse, während die Blitzlampe eine höhere Eindringtiefe aufweist und das Collagen erreicht. Die Erwärmung des Collagens durch den Er:YAG-Laser wird offensichtlich nicht als ausreichend angesehen. Aus diesem Grunde wird der Er:YAG-Laser in üblicher Weise lediglich zum Abtragen der oberen Hautschichten verwendet.

Der Erfindung liegt die Aufgabe zugrunde, eine Vorrichtung der eingangs genannten Art zu schaffen, die eine verbesserte Beseitigung von Hautschäden oder Hautbeeinträchtigungen, wie z.B. Narben, Falten und dergleichen, ermöglicht.

Diese Aufgabe wird durch die im Patentanspruch 1 angegebenen Merkmale gelöst.

Vorteilhafte Ausgestaltungen und Weiterbildungen der Erfindung ergeben sich aus den Unteransprüchen.

Bei der erfindungsgemäßen Vorrichtung erstreckt sich die Erwärmung ausgehend von der Hautoberfläche, in der die Absorption im wesentlichen stattfindet, über eine größere Schichtdicke unterhalb der Hautoberfläche, wobei bei einer bevorzugten Ausgestaltung der Erfindung auf einen Abtrag in vielen Fällen vollständig verzichtet werden kann. In anderen Fällen kann es sinnvoll sein, eine Kombination aus lokalem Abtrag (z.B. Faltenkämme) und großflächiger Erwärmung anzuwenden.

Die bisher anhand der Falten diskutierte Problematik trifft in ähnlicher Weise auch für die Behandlung von Narben zu. Grundsätzlich sollte hier die erneute Schädigung der Hautoberfläche minimiert werden. Auch hierbei ist eine gezielte wärmeinduzierte Schrumpfung von (Narben-) Kollagen möglich.

Als Lichtquelle können gemäß einer bevorzugten Ausgestaltung der Vorrichtung verschiedene Arten von Laserlichtquellen, wie z.B. ein Ho:YAG-, ein Er:YAG-, ein Er:YSGG-, ein Tm:YAG-, ein Co₂-Laser oder ein Nd:YAG-Laser verwendet werden, wobei dies lediglich einige Beispiele sind.

Bei allen Arten von Lichtquellen kann durch die erfindungsgemäße Impulssteuerung eine erhebliche Menge an Energie in das Gewebe unterhalb der Hautoberfläche eingebracht werden.

Hierbei wird die Energie so gewählt, daß sich kein Abtrag ergibt, daß sich jedoch durch Wärmeleitung eine ausreichende Erwärmung der tieferliegenden Kollagenschicht ergibt.

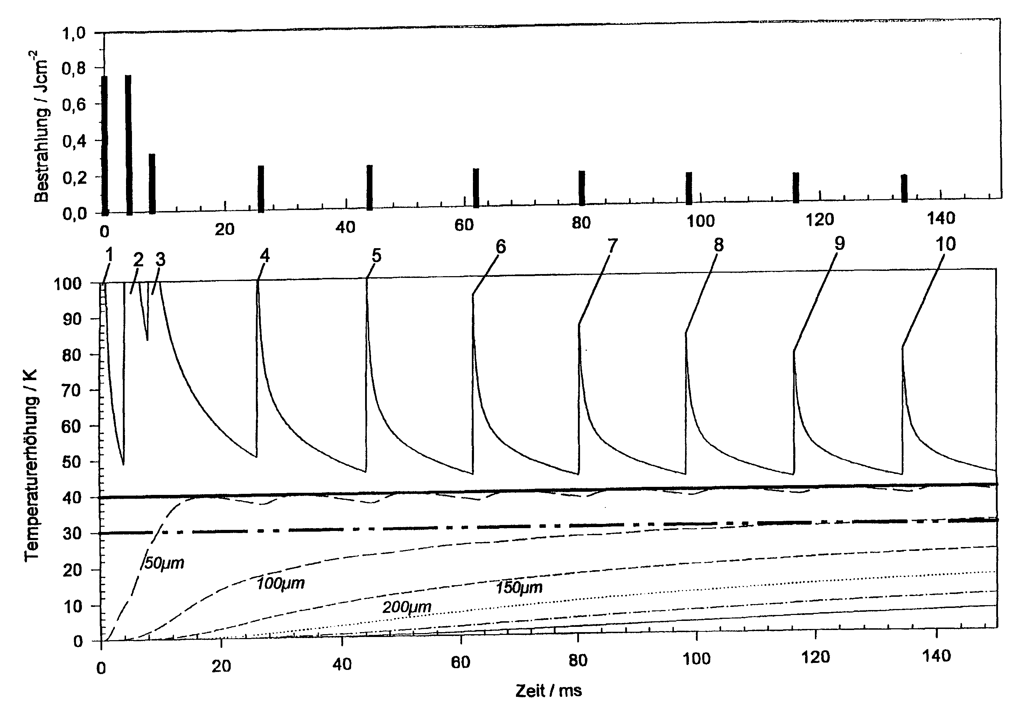

Die Erfindung wird im folgenden anhand eines in der Zeichnung dargestellten Ausführungsbeispiels näher erläutert.

In der Zeichnung ist in der oberen Hälfte eine Lichtimpulsfolge dargestellt mit so vorgewählten Energiedichten der Einzelimpulse, daß sich die im unteren Teil der Abbildung dargestellten Temperaturverläufe an der Hautoberfläche bzw. in den unter der Hautoberfläche liegenden Gewebeschichten in Tiefen von 50 µm, 100 µm, 150 µm und 200 µm im zeitlichen Verlauf ergeben. Die vertikale Skala gibt die Temperaturdifferenz ΔT in °K gegenüber der Hauttemperatur an.

Wie aus der Zeichnung zu erkennen ist, wird bei dem dargestellten Beispiel eine Folge von Laserimpulsen 1 bis 10 auf die Hautoberfläche appliziert, wobei die ersten Impulse der Folge, beispielsweise die Impulse 1 und 2 vorzugsweise eine höhere Energie, Energiedichte oder Leistung aufweisen, die jedoch unterhalb der Abtragschwelle liegen muß, um eine schnelle Erwärmung zunächst der Hautoberfläche und dann, zeitlich verzögert, durch Wärmeleitung eine Erwärmung der gewünschten darunterliegenden Gewebeschicht auf die Zieltemperatur zu erreichen. Wie aus der Kurve für eine Tiefe von 50 µm zu erkennen ist, steigt hierdurch die Temperatur in dieser Tiefe relativ schnell an und wird dann möglichst gleichförmig auf einer gewünschten Zieltemperaturdifferenz von beispielsweise 40°K gehalten. Wie zu erkennen ist, sinkt die Temperatur für die Kurve für 50 µm Tiefe nach Applikation der drei ersten Lichtimpulse wieder etwas ab und wird beispielsweise dann durch den bei 4 dargestellten Impuls wieder auf die gewünschte Grenztemperatur angehoben. Gleiches gilt auch für die nachfolgenden Impulse 5 bis 10, die jeweils ein Nachheizen auf die Zieltemperatur bewirken. Die auf die ersten Impulse folgenden Impulse der Impulsfolge können hierbei eine geringere Energie, bewirkt durch eine geringere Leistung und/oder Dauer, aufweisen, da die für die Aufrechterhaltung der gewünschten Zieltemperatur erforderliche Energie geringer wird. Die Temperatur in der Tiefe von 100 µm sowie in den tieferliegenden Schichten von 150 µm und 200 µm steigt hierbei kontinuierlich an und nähert sich einem gewünschten Grenzwert einer Temperaturdifferenz von beispielsweise 30°K für die Tiefe von 100 µm.

Gemäß einer vorteilhaften Ausgestaltung der Erfindung kann beispielsweise durch einen Oberflächentemperatursensor die Temperatur an der Hautoberfläche und ggf. in darunterliegenden Schichten überwacht werden, so daß eine entsprechende Impulssteuerung der einzelnen Impulse erfolgen kann, wobei z.B. sichergestellt wird, daß die vorgegebene Ziel-Temperatur z.B. einer irreversiblen thermischen Schädigung niemals überschritten wird.

Bei geringeren Anforderungen an eine schnelle Aufheizzeit kann natürlich genauso, ggf. unterstützt durch Messung der Oberflächentemperatur der Haut, ein gepulster Laser mit konstanter Impulsfolgefrequenz und Impulsenergie und Impulsenergie bzw. Impulsenergiedichte bzw. Impulsleistung unterhalb der Ablationsschwelle getaktet werden, so daß vergleichbare Temperaturerhöhungen erzielt werden können.

Die erfindungsgemäße Vorrichtung ermöglicht durch entsprechende Steuerung der Impulsfolgen und der Energien der einzelnen Impulse eine gezielte Behandlung der Haut, wobei entsprechend der gewünschten Anwendung diese Energien und Impulsdauern bzw. Impulsabstände gewählt werden können.

Die erfindungsgemäße Vorrichtung ist nicht allein für die thermische Veränderung von Kollagen-Fibrillen einsetzbar, sondern kann generell dazu dienen, physiologisch spezifische Temperaturerhöhungen hervorzurufen.

So ist es beispielsweise zur Erhöhung der Enzymaktivität in der Haut lediglich erforderlich, eine Temperaturerhöhung um wenige °K hervorzurufen. Zur Desaktivierung von Enzymen sollten unter der Hautoberfläche liegende Gewebeschichten auf eine Temperatur von etwa 43°C bis 55°C erwärmt werden. Für eine Schrumpfung von Kollagen ohne Koagulation sollten Temperaturen im Bereich von etwa 55°C bis 60°C und 70°C eingesetzt werden, für Koagulation der Temperaturbereich 70°C bis 100°C.

Falls die erfindungsgemäße Vorrichtung zur oberflächlichen Veränderung von Hartgeweben (Knochen, Zahnschmelz) eingesetzt werden soll, so sollte die Temperaturerhöhung einige 100K betragen.

Falls dies erwünscht ist, kann auf die in der Zeichnung dargestellte Impulsfolge ein nachgeschalteter Abtragimpuls mit höherer Leistung folgen.

Wenn die erfindungsgemäße Vorrichtung zum Abtrag von malignem oder bakteriell oder viral kontaminiertem Gewebe verwendet werden soll, so verringert sich durch die Möglichkeit, vor dem Abtragen eine Koagulierung vorzunehmen, die Gefahr, lebende Tumorzellen, Bakterien oder Viren zu verschleppen.

Bei einem speziellen, der Zeichnung entsprechenden Beispiel wurde eine Anzahl von Impulsen eines Er:YAG-Lasers mit einer Gesamtenergie von etwa 100 mJ verwendet (vgl. Tabelle). Die ersten drei Impulse enthalten hierbei mit insgesamt 56 mJ mehr als die Hälfte der Gesamtenergie, wobei diese Impulse relativ schnell aufeinander folgen. Dies führt zu einer schnellen Erwärmung der Oberfläche auf die zugelassene Zieltemperaturerhöhung ΔT = 40°K in 50 µm Tiefe. Die nachfolgenden Impulse 4 bis 10 dienen lediglich dazu, diese Temperatur zu stabilisieren. Zu dieser Stabilisierung sind in der Folge immer geringere Einzelimpulsenergien erforderlich, wobei zwischen den einzelnen Impulsen verhältnismäßig lange Pausen vorliegen können. Auf diese Weise werden dann nahezu stabile Gradienten in tieferen Gewebeschichten erzielt, wie sich dies aus den Kurven für eine Tiefe von 100 µm bzw. 150 µm und 200 µm am Ende der zehn Impulse erkennen läßt.

**TABELLE**

| Pulsnummer | 1 | 2 | 3 | 4 | 5 | 6 | 7 | 8 | 9 | 10 |
|---|---|---|---|---|---|---|---|---|---|---|
| Energie / mJ | 23,3 | 23,3 | 9,4 | 7,2 | 6,9 | 6,0 | 5,3 | 4,7 | 4,4 | 4,4 |
| Bestrahlung/Jcm⁻² | 0,74 | 0,74 | 0,30 | 0,23 | 0,22 | 0,19 | 0,17 | 0,15 | 0,14 | 0,13 |
| zeitlicher Abstand zum vorangehenden Puls / ms | - | 4 | 4 | 18 | 18 | 18 | 18 | 18 | 18 | 18 |

Dieser Laser und die Impulsfolge stellt lediglich ein bevorzugtes Ausführungsbeispiel ohne jede Beschränkung dar.

Es besteht beispielsweise auch die Möglichkeit, einen Laser mit hoher Impulsfrequenz, beispielsweise mit 50 Hz, zu verwenden, der gegebenenfalls zusätzlich durch einen die Temperatur der Hautoberfläche messenden Sensor gesteuert wird.

## Patentansprüche

1. Vorrichtung zum oberflächlichen Erwärmen von Haut, mit einer impulsförmig ansteuerbaren. Lichtquelle und einer Steuereinheit zur Steuerung der Lichtquelle; die die Lichtquelle derart ansteuert, daß Folgen von Lichtimpulsen jeweils mit vorgegebener Dauer und Bestrahlungsstärke geliefert werden, **dadurch gekennzeichnet, daß** die Lichtquelle Lichtimpulse mit einer derartigen Wellenlänge liefert, daß die Lichtimpulse hauptsächlich in einem Bereich an der Hautoberfläche absorbiert werden, der oberhalb einer Gewebetiefe von 50µm liegt, daß jede Folge einen ersten Teil umfaßt, der eine schnelle Erwärmung dieses Bereiches der Hautoberfläche und dann zeitlich verzögert durch Wärmeleitung eine Erwärmung von unter diesem Bereich der Hautoberfläche liegenden Gewebebereichen auf eine vorgegebene Zieltemperatur bewirkt, die unterhalb der Temperatur liegt, bei der ein Gewebeabtrag auftritt, und einen nachfolgenden Teil umfaßt, der ein Oszillieren der Temperatur der Hautoberfläche und/oder des unter der Hautoberfläche liegenden Gewebebereichs um die Zieltemperatur bewirkt, wobei diese Temperatur ebenfalls unterhalb einer einen Abtrag hervorrufenden Temperatur liegt.

2. Vorrichtung nach Anspruch 1,
**dadurch gekennzeichnet, daß** die Vorrichtung eine Meßeinrichtung zur Messung der Oberflächentemperatur aufweist, und daß das Ausgangssignal der Meßeinrichtung die Steuereinheit ansteuert.

3. Vorrichtung nach Anspruch 2,
**dadurch gekennzeichnet, daß** die Meßeinrichtung eine radiometrische Meßeinrichtung ist.

4. Vorrichtung nach einem der Ansprüche 1 bis 3,
**dadurch gekennzeichnet, daß** die Steuereinheit die Lichtquelle derart steuert, daß auf den nachfolgenden Teil der oder jeder Folge ein Impuls höherer Energie, Energiedichte oder Leistung folgt, der eine Abtragung von Gewebe bewirkt.

5. Vorrichtung nach Anspruch 4,
**dadurch gekennzeichnet, daß** der erste Teil der Folge ein schnelles Aufheizen auf eine Zieltemperatur zwischen der Koagulations- und der Abtragstemperatur bewirkt, während der nachfolgende Teil der Impulsfolge eine Vergrößerung der Koagulationszone bewirkt, und daß der Abtragimpuls so bemessen ist, daß ein kleiner Saum des koagulierten Gewebes bestehen bleibt.

6. Vorrichtung nach einem der Ansprüche 1 bis 3,
**dadurch gekennzeichnet, daß** mit dem ersten Teil und dem nachfolgenden Teil der Impulsfolge eine Temperaturerhöhung auf eine Temperatur von weniger als 43°C erreicht wird, die eine Erhöhung der Enzymaktivität in der Haut bewirkt.

7. Vorrichtung nach einem der Ansprüche 1 bis 3,
**dadurch gekennzeichnet, daß** mit dem ersten Teil und dem nachfolgenden Teil der Impulsfolge eine Temperaturerhöhung auf eine Temperatur im Bereich von 43°C bis 55°C erreicht wird, die eine Desaktivierung von Enzymen bewirkt.

8. Vorrichtung nach einem der Ansprüche 1 bis 3,
**dadurch gekennzeichnet, daß** mit dem ersten Teil und dem nachfolgenden Teil der Impulsfolge eine Temperaturerhöhung auf eine Temperatur im Bereich von 55°C bis 70°C erreicht wird, die eine Schrumpfung von Kollagen ohne Koagulation bewirkt.

9. Vorrichtung nach einem der Ansprüche 1 bis 3,
**dadurch gekennzeichnet, daß** die Lichtquelle ein Er:YAG- oder ER:YSGG-Lasermaterial verwendet.

10. Vorrichtung nach einem der Ansprüche 1 bis 3,
**dadurch gekennzeichnet, daß** die Lichtquelle ein CO₂-Laser ist.

## Claims

1. Apparatus for the superficial heating of skin comprising a light source which can be actuated in pulse form and a control unit for controlling the light source which actuates the light source in such a way that sequences of light pulses each of a predetermined duration and irradiation strength are delivered, **characterised in that** the light source delivers light pulses of a wavelength such that the light pulses are absorbed primarily in a region at the surface of the skin which is above a tissue depth of 50 µm, that each sequence comprises a first part which causes rapid heating of said region of the surface of the skin and then in time-delayed relationship by heat conduction heating of tissue regions below said region of the surface of the skin to a predetermined target temperature which is below the temperature at which tissue removal occurs, and a subsequent part which causes oscillation of the temperature of the surface of the skin and/or of the tissue region below the surface of the skin about the target temperature, wherein said temperature is also below a temperature causing removal.

2. Apparatus according to claim 1 **characterised in that** the apparatus has a measuring device for measuring the surface temperature and that the output signal of the measuring device actuates the control unit.

3. Apparatus according to claim 2 **characterised in that** the measuring device is a radiometric measuring device.

4. Apparatus according to one of claims 1 to 3 **characterised in that** the control unit controls the light source in such a way that the subsequent part of the or each sequence is followed by a pulse of higher energy level, energy density or power, which causes removal of tissue.

5. Apparatus according to claim 4 **characterised in that** the first part of the sequence causes rapid heating up to a target temperature between the coagulation and the removal temperatures while the subsequent part of the pulse sequence causes an increase in the size of the coagulation zone, and that the removal pulse is of such dimension that a small edge of the coagulated tissue remains.

6. Apparatus according to one of claims 1 to 3 **characterised in that** the first part and the subsequent part of the pulse sequence provide for a temperature increase to a temperature of less than 43°C which causes an increase in enzyme activity in the skin.

7. Apparatus according to one of claims 1 to 3 **characterised in that** the first part and the subsequent part of the pulse sequence provide for a temperature increase to a temperature in the range of 43°C to 55°C which causes deactivation of enzymes.

8. Apparatus according to one of claims 1 to 3 **characterised in that** the first part and the subsequent part of the pulse sequence provide for a temperature increase to a temperature in the range of 55°C to 70°C which causes shrinkage of collagen without coagulation.

9. Apparatus according to one of claims 1 to 3 **characterised in that** the light source uses an Er:YAG or Er:YSGG laser material.

10. Apparatus according to one of claims 1 to 3 **characterised in that** the light source is a CO₂ laser.

## Revendications

1. Dispositif pour chauffer superficiellement la peau, comportant une source de lumière pouvant être commandée de façon impulsionnelle et une unité de commande servant à commander la source de lumière et qui commande ladite source de lumière de telle sorte que des suites d'impulsions de lumière ayant respectivement une durée prédéterminée et une intensité de rayonnement prédéterminée sont délivrées, **caractérisé en ce que** la source de lumière délivre des impulsions de lumière ayant une longueur d'onde telle que les impulsions de lumière sont absorbées principalement au niveau de la surface de la peau dans une zone qui se situe au-dessus d'une profondeur de tissu de 50 µm, que chaque suite comporte une première partie qui provoque un chauffage rapide de cette zone de la surface de la peau et ensuite, d'une manière retardée, par conduction thermique un chauffage de zones de tissu situées au-dessous de cette zone de la surface de la peau, à une température de consigne prédéterminée, qui est inférieure à la température, pour laquelle apparaît un enlèvement du tissu, et une partie suivante, qui provoque une oscillation de la température de la surface de la peau et/ou de la zone de tissu située au-dessous de la surface de la peau, autour de la température de consigne, cette température étant également inférieure à une température provoquant un enlèvement.

2. Dispositif selon la revendication 1, **caractérisé en ce que** le dispositif comporte un dispositif de mesure pour mesurer la température superficielle, et que le signal de sortie du dispositif de mesure commande l'unité de commande.

3. Dispositif selon la revendication 2, **caractérisé en ce que** le dispositif de mesure est un dispositif de mesure radiométrique.

4. Dispositif selon l'une des revendications 1 à 3,
**caractérisé en ce que** l'unité de commande de la source de lumière effectue une commande de telle sorte qu'à la partie suivante de la ou de chaque suite succède une impulsion possédant une énergie plus élevée, une densité d'énergie plus élevée ou une puissance plus élevée, qui réalise un enlèvement de tissu.

5. Dispositif selon la revendication 4, **caractérisé en ce que** la première partie de la suite provoque un échauffement rapide à une température de consigne située entre la température de coagulation et la température d'enlèvement, tandis que la partie suivante de la suite d'impulsions provoque un agrandissement de la zone de coagulation et que l'impulsion d'enlèvement est dimensionnée de telle sorte qu'il subsiste un faible filet de jonction du tissu coagulé.

6. Dispositif selon l'une des revendications 1 à 3, **caractérisé en ce qu'**avec la première partie et la partie suivante de la suite d'impulsions on obtient un accroissement de température à une température inférieure à 43°C, qui réalise un accroissement de l'activité enzymatique dans la peau.

7. Dispositif selon l'une des revendications 1 à 3, **caractérisé en ce qu'**avec la première partie et la partie suivante de la suite d'impulsions, on obtient un accroissement de température à une température dans la gamme de 43°C à 55°C, qui réalise une désactivation d'enzymes.

8. Dispositif selon l'une des revendications 1 à 3, **caractérisé en ce qu'**avec la première partie et la partie suivante de la suite d'impulsions on obtient un accroissement de température à une température dans la gamme de 55°C à 70°C, qui réalise une contraction du collagène sans coagulation.

9. Dispositif selon l'une des revendications 1 à 3, **caractérisé en ce que** la source de lumière utilise un matériau laser Er:YAG ou Er:YSGG.

10. Dispositif selon l'une des revendications 1 à 3, **caractérisé en ce que** la source de lumière est un laser au CO₂.
